Europäisches Patentamt

European Patent Office

Office européen des brevets

(11) Publication number: **0 271 219 B1**

## EUROPEAN PATENT SPECIFICATION

(45) Date of publication of patent specification: 29.07.92

(51) Int. Cl.⁵: **C07J 73/00**, A61K 31/435, A61K 31/58

(21) Application number: **87309949.3**

(22) Date of filing: **11.11.87**

The file contains technical information submitted after the application was filed and not included in this specification

(54) **Topical pharmaceutical composition containing 17-beta-methoxycarbonyl-4-methyl-4-aza-5-alpha-androst-1-en-3-one.**

(30) Priority: **20.11.86 US 932549**

(43) Date of publication of application:
**15.06.88 Bulletin 88/24**

(45) Publication of the grant of the patent:
**29.07.92 Bulletin 92/31**

(84) Designated Contracting States:
**AT BE CH DE ES FR GB IT LI LU NL SE**

(56) References cited:
**EP-A- 0 004 949**
**EP-A- 0 155 096**

**JOURNAL OF MEDICINAL CHEMISTRY, vol. 29, no. 11, November 1986, pages 2298-2315, American Chemical Society, Washington, D.C., US; G.H. RASMUSSON et al.: "Azasteroids: Structure-activity relationships for inhibition of 5alpha-reductase and of androgen receptor binding"**

(73) Proprietor: **MERCK & CO. INC.**
**126, East Lincoln Avenue P.O. Box 2000**
**Rahway New Jersey 07065-0900(US)**

(72) Inventor: **Rasmusson, Gary H.**
**155 Park Place**
**Watchung New Jersey 07060(US)**
Inventor: **Reynolds, Glenn F.**
**252 Edgewood Avenue**
**Westfield New Jersey 07090(US)**

(74) Representative: **Hesketh, Alan, Dr.**
**European Patent Department Merck & Co., Inc. Terlings Park Eastwick Road Harlow Essex, CM20 2OR(GB)**

Rank Xerox (UK) Business Services

## Description

The present invention is concerned with a topical pharmaceutical composition comprising 17β-methoxycarbonyl-4-methyl-4-aza-5α-androst-1-en-3-one.

It is well known in the art that certain undesirable physiological manifestations, such as acne vulgaris, seborrhea, female hirsutism, and male pattern baldness and benign prostatic hypertrophy, are the result of hyperandrogenic stimulation caused by an excessive accumulation of testosterone or similar androgenic hormones in the metabolic system. Early attempts to provide a chemotherapeutic agent to counter the undesirable results of hyperandrogenicity resulted in the discovery of several steroidal antiandrogens having undesirable hormonal activities of their own. The estrogens, for example, not only counteract the effect of the androgens but have a feminizing effect as well. Non-steroidal antiandrogens have also been developed, for example, 4'-nitro-3'-trifluoromethylisobutyranilide. See Neri et al., Endo., Vol. 91, No1 2 (1972). However, these products, though devoid of hormonal effects, are peripherally active, competing with the natural androgens for receptor sites, and hence have a tendency to feminize a male host or the male fetus of a female host.

It more recently became known in the art that the principal mediator of androgenic activity in some target organs is 5α-dihydrotestosterone, and that it is formed locally in the target organ by the action of testosterone-5α-reductase. It therefore has been postulated and demonstrated that inhibitors of testosterone-5α-reductase will serve to prevent or lessen symptoms of hyperandrogenic stimulation. Nayfeh et al., Steroids, 14, 269 (1969) demonstrated in vitro that methyl 4-androsten-3-one-17β-carboxylate was a testosterone-5α-reductase inhibitor. Then Voigt and Hsia, Endocrinology, 92, 1216 (1973), Canadian Pat. No. 970,692, demonstrated that the above ester and the parent free acid, 4-androsten-3-one-17β-carboxylic acid are both active inhibitors of testosterone-5α-reductase in vitro. They further demonstrated that topical application of either testosterone or5α-dihydrotestosterone caused enlargement of the female hamster flank organ, or androgen dependent sebaceous structure. However, concomitant administration of 4-androsten-3-one-17β-carboxylic acid or its methyl ester inhibited the response elicited by testosterone but did not inhibit the response elicited by 5α-dihydrotestosterone. These results were interpreted as indicating that the compounds were antiandrogenic by virtue of their ability to inhibit testosterone-5α-reductase.

A number of 4-aza steroid compounds are known. See, for example, U.S. Pat. Nos. 2,227,876; 3,239,417; 3,264,301; and 3,285,918; French Pat. No. 1,465,544; Doorenbos and Solomons, J. Pharm. Sci. 62, 4, pp. 638-640 (1973); Doorenbos and Brown, J. Pharm. Sci., 60, 8, pp. 1234-1235 (1971); and Doorenbos and Kim, J. Pharm. Sci., 63, 4, pp. 620-622 (1974).

In addition U.S. Patent 4,377,584 and 4,220,775 and EP-A-4949 of Rasmusson et al. describe a group of 4-aza-5α-17β-substituted-5α-androsten-3-ones which are useful in the treatment of hyperandrogenic conditions. Recently, a number of investigators from Merck & Co., Inc. have published regarding the biological activity of 5α-reductase inhibitors. See for example, Rasmusson et al., J. Med. Chem. 29: 2298 (1986), Brooks, et al., The Prostate 9: 65-75 (1986), Liang et al., Endrocrinology 117, No. 2, pp. 571-579 (1985), Rasmusson et al., J. Med. Chem. 27: 1690-1701 (1984), Liang et al., J. Biol. Chem. 259, No. 2, pp. 734-739 (1984). Rasmusson, et al, J. Med Chem, 29, 2298-2315 (1986) discloses inter alia 17β-methoxycarbonyl-4-methyl-4-aza-5α-androst-1-en-3-one.

The present invention is concerned with a topical pharmaceutical composition comprising 17β-methoxycarbonyl-4-methyl-4-aza-5α-androst-1-en-3-one. The preparation of this compound is described in Rasmussen et al., J. Med Chem, 29, 2298-2315 (1986).

These compositions are useful for treating the hyperandrogenic conditions of acne, seborrhea, hirsutism and male pattern baldness.

For the treatment of acne vulgaris, seborrhea, female hirsutism, 17β-methoxycarbonyl-4-methyl-4-aza-5α-androst-1-en-3-one is administered in the formula of pharmaceutical composition comprising the active compound in combination with a pharmacologically acceptable carrier adapted for topical administration. These topical pharmaceutical compositions may be in the form of a cream, ointment, gel or aerosol formulation adapted for application to the skin. These topical pharmaceutical compositions containing said compound ordinarily include about 0.1% to 15%, preferably about 5%, of the active compound, in admixture with about 95% of vehicle.

## Claims

**Claims for the following Contracting States :**
**AT, BE, CH, DE, FR, GB, IT, LI, LU, NL, SE**

1.  A topical pharmaceutical composition comprising 17β-methoxycarbonyl-4-methyl-4-aza-5α-androst-1-en-3-one.

2.  The use of 17β-methoxycarbonyl-4-methyl-4-aza-5α-androst-1-en-3-one for the preparation for a medicament useful for the topical treatment of the hyperandrogenic conditions of acne vulgaris, seborrhea, hirsutism and male pattern baldness.

**Claim for the following Contracting State : ES**

1. A process for the preparation of a topical pharmaceutical composition comprising mixing a pharmaceutically acceptable carrier and a therapeutically effective amount of $17\beta$-methoxycarbonyl-4-methyl-4-aza-$5\alpha$-androst-1-en-3-one.

**Revendications**
**Revendications pour les Etats contractants suivants : AT, BE, CH, DE, FR, GB, IT, LI, LU, NL, SE**

1. Composition pharmaceutique topique qui comprend la $17\beta$-méthoxycarbonyl-4-méthyl-4-aza-$5\alpha$-androst-1-en-3-one.

2. Utilisation de la $17\beta$-méthoxycarbonyl-4-méthyl-4-aza-$5\alpha$-androst-1-en-3-one pour la préparation d'un médicament utile pour le traitement topique des états hyperandrogènes de l'acné vulgaire, de la séborrhée, de l'hirsutisme et de l'alopécie de modèle masculin.

**Revendication pour l'Etat contractant suivant : ES**

1. Procédé pour la préparation d'une composition pharmaceutique topique qui comprend le mélange d'un véhicule pharmaceutiquement acceptable et d'une quantité thérapeutiquement efficace de la $17\beta$-méthoxycarbonyl-4-méthyl-4-aza-$5\alpha$-androst-l-en-3-one.

**Patentansprüche**
**Patentansprüche für folgende Vertragsstaaten : AT, BE, CH, DE, FR, GB, IT, LI, LU, NL, SE**

1. Topische pharmazeutische Zusammensetzung, welche $17\beta$-Methoxycarbonyl-4-methyl-4-aza-$5\alpha$-androst-1-en-3-on enthält.

2. Verwendung von $17\beta$-Methoxycarbonyl-4-methyl-4-aza-$5\alpha$-androst-1-en-3-on zur Herstellung eines Medikaments, das zur topischen Behandlung von hyperandrogenen Zuständen von Akne vulgaris, Seborrhö, Hirsutismus und Haarausfall nach männlichem Muster geeignet ist.

**Patentanspruch für folgenden Vertragsstaat : ES**

1. Verfahren zur Herstellung einer topischen pharmazeutischen Zusammensetzung durch Mischen eines pharmazeutisch annehmbaren Trägers und einer therapeutisch wirksamen Menge von $17\beta$-Methoxycarbonyl-4-methyl-4-aza-$5\alpha$-androst-1-en-3-on.